(19) Europäisches Patentamt
European Patent Office
Office européen des brevets

(11) Publication number: **0 130 069 B1**

(12) **EUROPEAN PATENT SPECIFICATION**

(45) Date of publication of patent specification: **12.12.90**

(21) Application number: **84304247.4**

(22) Date of filing: **22.06.84**

(51) Int. Cl.5: **C 07 D 471/04,**
C 07 D 491/147,
A 61 K 31/435 // C07D207/06,
C07D207/26, C07D207/40
,(C07D471/04, 221:00, 209:00)

(54) **Hexahydropyrrolo (2,1-a) isoquinoline derivatives.**

(30) Priority: **23.06.83 US 507250**

(43) Date of publication of application:
**02.01.85 Bulletin 85/01**

(45) Publication of the grant of the patent:
**12.12.90 Bulletin 90/50**

(84) Designated Contracting States:
**AT BE CH DE FR GB IT LI NL SE**

(56) References cited:
**FR-A-2 081 412**
**GB-A-1 153 670**

**J. Med.Chem, 27, 943, 1984**

(73) Proprietor: **McNeilab, Inc.**
**Springhouse Pennsylvania 19477 (US)**

(72) Inventor: **Maryanoff, Bruce E.**
**P.O. Box 56 Aquetong Road**
**New Hope Pennsylvania 18938 (US)**

(74) Representative: **Jones, Alan John et al**
**CARPMAELS & RANSFORD 43 Bloomsbury Square**
**London, WC1A 2RA (GB)**

**EP 0 130 069 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel chemical compounds, which are derivatives of 1,2,3,5,6,10b-hexahydro-pyrrolo[2,1-a]-isoquinolines, and non-toxic, pharmaceutically-acceptable salts thereof. It also relates to novel synthetic intermediates and processes leading to said compounds.

GB—A—1 153 670 (Siphar) discloses compounds of the formula:

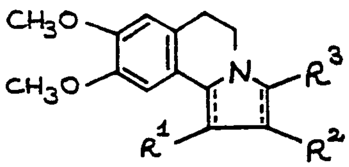

in which the pyrrole ring may be unsaturated or saturated, $R^1$ is a hydrogen atom or a carboxyl or esterified carboxyl group, $R^2$ is an alkyl, cycloalkyl, aryl, carboxyl, esterified carboxyl, carboxymethyl, esterified carboxymethyl, carboxamido or substituted carboxamido group, $R^3$ is a hydrogen atom or a carboxyl or esterified carboxyl group, or $R^2$ and $R^3$ when taken together may be the anhydride group of the corresponding dicarboxylic acid. These compounds are said to have hypotensive, sympathicolytic and pschotropic properties and are indicated as being useful for the treatment of malfunctions of the cardiocirculatory system and also of the nervous system.

FR—A—2 081 412 (Roche) discloses compounds of the formula:

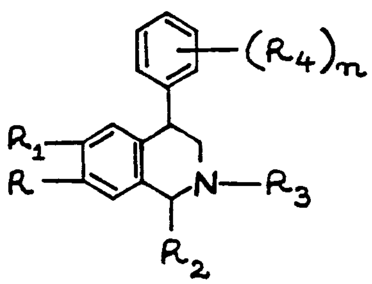

wherein R represents hydroxy or lower alkoxy, $R_1$ represents hydrogen, hydroxy or lower alkoxy, or R and $R_1$ together represent methylenedioxy, $R_2$ represents hydrogen or lower alkyl, $R_3$ represents lower alkyl or lower aralkyl, $R_4$ represents halogen, nitro, or mono- or di-lower alkyl substituted amino, and n is 1 or 2. These compounds are shown to have antidepressive properties.

According to the present invention there are provided compounds of the general formula I

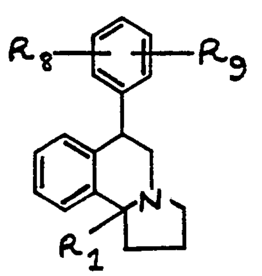

I

wherein:

$R_1$ is hydrogen or $C_1$—$C_4$ alkyl;

$R_8$ and $R_9$ are independently hydrogen, $C_1$—$C_4$ alkyl, perfluoro $C_1$—$C_4$ alkyl, hydroxy, $C_1$—$C_4$ alkoxy, carb ($C_1$—$C_4$) alkoxy, $C_1$—$C_5$ acylamino, benzoylamino, cyano, carboxamido, $C_1$—$C_5$ acyl, $C_1$—$C_4$ alkylthio, $C_1$—$C_4$ alkylsulfonyl, nitro, amino, $C_1$—$C_4$ alkyl- or di($C_1$—$C_4$) alkyl-amino, or halogen;

including diastereomers and the nontoxic, pharmaceutically-acceptable acid addition salts thereof.

Preferably, $R_8$ and $R_9$ are independently hydrogen, $C_1$—$C_6$ alkyl, perfluoro ($C_1$—$C_4$) alkyl, hydroxy, $C_1$—$C_4$ alkoxy, carb ($C_1$—$C_4$) alkoxy, $C_1$—$C_5$ acylamino, $C_1$—$C_5$ acyl, $C_1$—$C_4$ alkylthio, $C_1$—$C_4$ alkylsulfonyl, nitro, amino, $C_1$—$C_4$ alkyl- or di($C_1$—$C_4$) alkyl-amino, or halogen.

The present invention also provides a pharmaceutical composition comprising a compound according to the invention as defined above in combination with a pharmaceutically acceptable diluent or carrier.

The present invention also comprises a compound or a composition according to the invention as defined above for use in treating depression in a mammal.

As used herein, the terms "alkyl", "alkoxy", "alkylthio", "alkylsulfonyl" and "perfluoroalkyl" refer to straight- or branched-chain carbon skeletons, within the carbon atom limits defined. The term halo (or halogen) is generic for fluorine, chlorine, bromine or iodine.

Each formula I compound describes and comprises diastereomeric substances, themselves pairs of enantiomers. The diastereomers, isolated in their pure form, may differ in biological activity. The compounds of formula I constitute valuable therapeutic agents by their possession of psychotropic activity, particularly antidepressant activity.

The various diastereomers of each formula I compound are distinguished herein using the nomenclature recommended by Chemical Abstracts for representing the relative configuration of diastereomers of fused-ring compounds (α/β nomenclature). This requires that the stereocenter corresponding to the lowest numbered atom in the ring system (numbered according to convention) be designated α and the remaining stereocenters be labelled α or β relative to the first-assigned center. For example:

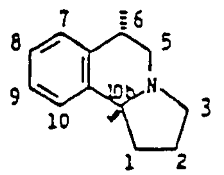
6α, 10bα

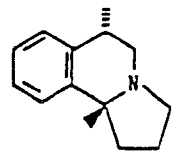
6α, 10bβ

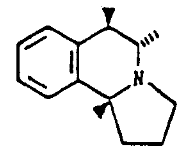
5α, 6β, 10bβ

The new compounds of the present invention can be prepared by various methodologies, known to those skilled in the art of organic chemistry. Some examples of important synthetic routes are illustrated below. These are exemplary methods and should not be taken as exhaustive. For the sake of simplicity, the routes are depicted without substituents on the aliphatic chains or aromatic rings, but this should not be construed to limit necessarily the scope of the synthetic process.

The $R_1$ group of formula I is depicted in the following routes as a specific moiety, e.g. hydrogen or methyl, for the purpose of specific exemplification. However, the other $R_1$ possible moieties may be used in their place by choice of a different starting material.

## (A) Keto-Acid, Acyliminium-Ion Route:

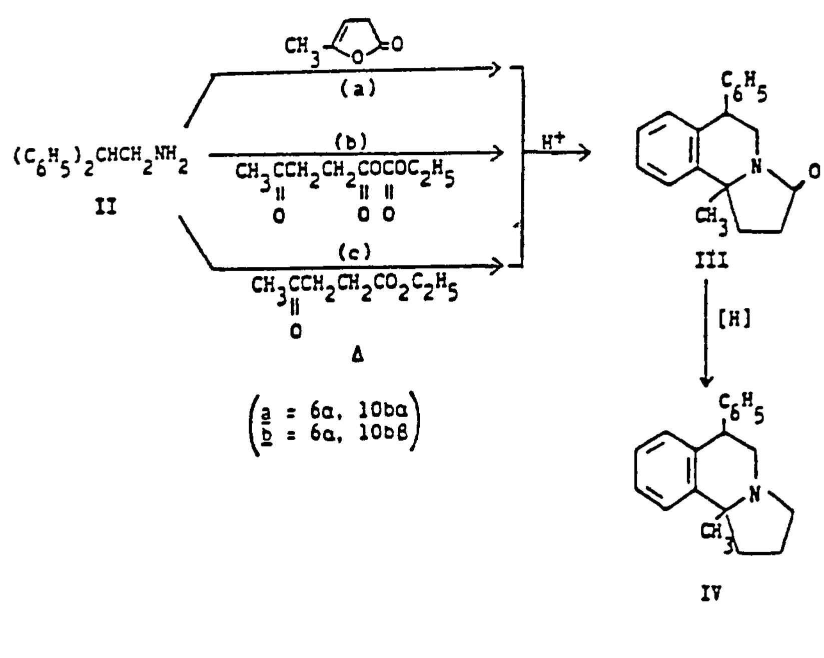


II, A, III, IV

(B) Imide, Acyiminium-Ion Route:

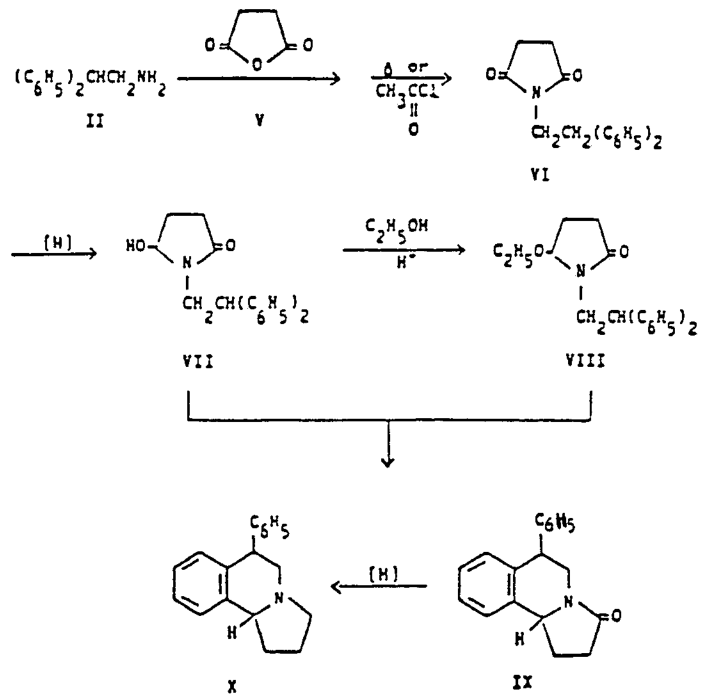


II V VI VII VIII X IX

(C) Mandelic-Acid Route:

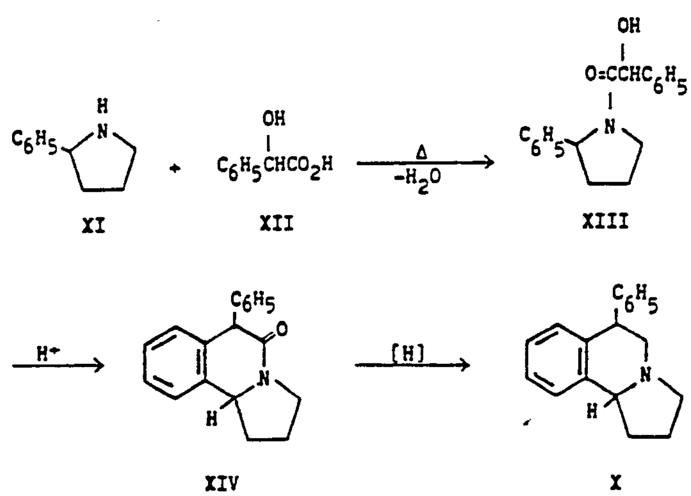


XI XII XIII XIV X

(D) Styrene-Oxide Route:

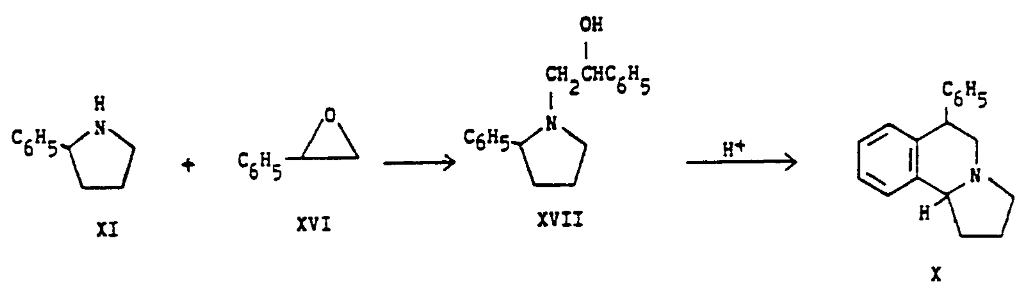


XI XVI XVII X

(E) Butyrolactone Route:

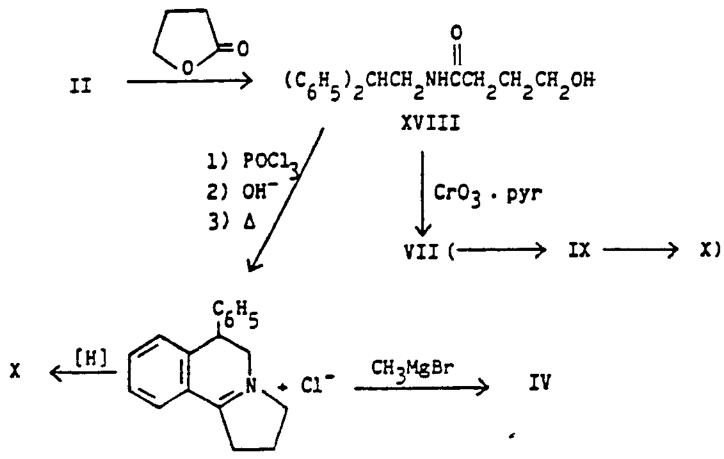


XIX

The keto-acid route (A) entails condensation of an arylethylamine, such as II, with (a) α-angelicalactone (or congeners thereof) in an inert solvent, such as methylene chloride or ethyl acetate, around ambient temperature, or (b) the mixed carbonic anhydride of levulinic acid (or congeners thereof) in an inert solvent, such as tetrahydrofuran, ethyl ether or toluene, around 0°C, or (c) a simple ester of levulinic acid (or congeners thereof) without solvent at elevated temperatures around 175—225°C. The adduct (not shown) is then cyclized with an acid catlayst to a lactam intermediate, such as III. The acid catalyst must be strong when cyclization is to take place on an aromatic ring not bearing a good electron-donating substituent (e.g., methoxy) ortho or para to the site of ring closure: e.g., polyphosphoric acid, liquid hydrogen fluoride, pyridinium polyhydrogen fluoride, or trifluoromethanesulfonic acid. When an electron-donating group is present in a proper orientation, cyclization can be effected with weaker acid catalysts, e.g., ethanolic hydrogen chloride or trifluoroacetic acid, and cyclization will preferentially occur on the ring having the greater activation in a competitive situation. The final product, such as IV, is then obtained by reduction of the amide functionality, employing aluminium or boron hydrides, e.g., lithium aluminum hydride or borane-tetrahydrofuran.

The imide route (B) is analogous to the keto-acid route, but yields 10b-unsubstituted derivatives. This route involves reaction of an arylethylamine, such as II, with succinic anhydride V (or congeners thereof) in an inert solvent, such as methylene chloride, ethyl acetate, or tetrahydrofuran, around ambient temperature. The amide-acid intermediate (not shown) is converted to a succinimide, such as VI, by heating without solvent at 150—200°C, or by addition of acetylchloride to the original mixture and heating at 40—70°C. The succinimide is then reduced with sodium borohydride in ethanol, as described by Speckamp (J. C. Hubert, et al., Tetrahedron, 31, 1437 (1975)), or by diisobutylaluminum hydride, as described by Hart (D. J. Hart and K. Kanai, J. Org. Chem., 47, 1555 (1982)). One may employ either a hydroxy or ethoxy species, such as VII or VIII, in an acid-catalyzed cyclization as described above for the keto-acid route, affording a lactam such as IX. The lactam is then reduced to a target amine, such as X, as described above for the keto-acid route.

The mandelic-acid route (C) entails thermal condensation (140—170°C) of a 2-arylpyrrolidine, such as XI, with a mandelic acid, such as XII, with continuous removal of water, to give a mandelamide, such as XIII. The mandelamide is cyclized to a lactam, such as XIV, with a strong acid catalyst, e.g., polyphosphoric acid, sulfuric acid, or liquid hydrogen fluoride. The lactam is reduced preferably with a Lewis acid-type hydride reagent such as with borane-tetrahydrofuran to yield a target amine such as X.

The styrene-oxide route (D) involves condensation of a 2-arylpyrrolidine, such as XI, with a styrene oxide, such as XVI, in refluxing ethanol or in sulfolane at 140—170°C, to give an amino alcohol, such as XVII. The amino alcohol is cyclized by heating with an acid catalyst, e.g., polyphosphoric acid, 48% hydrobromic acid, or sulfuric acid, to furnish a product amine, such as X.

The butyrolactone route (E) is based on reaction of an arylethylamine, such as II, with butyrolactone (or congeners thereof), without solvent around 100°C, to give an amido alcohol, such as XVIII. The amido alcohol can be converted to a key intermediate of the imide route, such as VII, for transformation into target compounds of formula (I), as indicated in route (B), by careful oxidation with chromium trioxide in pyridine. Alternatively, the amido alcohol can be converted to an iminium salt, such as XIX, with phosphorus oxychloride in toluene at 110°C, followed by treatment of the toluene-insoluble material with aqueous sodium hydroxide and heating in toluene solution. The iminium salt can be reduced to a target amine, such as X, with $NaBH_4$ or $LiAlH_4$. The iminium salt can also be reacted with an organometallic reagent, such as an organolithium or organomagnesium (Grignard) compound to afford 10b-substituted target amines of formula (I). For example, a Grignard reaction with methyl magnesium bromide, in ether or tetrahydrofuran, leads to a 10b-methyl compound of formula (I), such as IV.

The cyclization reactions in routes (A)—(E) afford mixtures of diastereomers. In certain instances, product mixtures may be highly enriched in specific diastereomers. The diastereomers may be separated and purified by standard techniques known to those skilled in the art of organic chemistry, such as fractional crystallization or liquid chromatography of free bases, or fractional crystallization of acid-addition salts.

Diastereomers may be interconverted by base-induced exchange of protons at the 6 and/or 10b positions. Specifically, heating of lactam diasteromers related to formula (I) compounds possessing 6 and/or 10b protons in aqueous dimethylsulfoxide around 100—150°C in the presence of an alkali metal carbonate, such as $K_2CO_3$, for 1—1000 hours can give rise to equilibrium mixtures of diastereomers. Also, heating of amine diastereomers of formula (I) possessing 6 and/or 10b protons in aqueous dimethylsulfoxide around 80—150°C in the presence of an alkali metal hydroxide such as NaOH, for 1—60 hours, can give rise to equilibrium mixtures of diastereomers. Such equilibration tactics can enhance the proportions of minor diastereomers in comparison to the original product mixtures from cyclization. In appropriate instances, the equilibration method can alter relative configuration between the 6 stereocenter in relation to the 2, 5, and 10b stereocenters for compounds of general formula (I) bearing a 6-position proton, between the 10b stereocenter and the 2, 5 and 6 stereocenters for compounds of formula (I) bearing a 10b-position proton, but not between the 2 and 5 stereocenters in compounds of formula (I).

Another means of changing the original diastereomeric composition of formula (I) compounds bearing a 10b-position proton involves oxidation with mercuric acetate, followed by direct reduction of the intermediate iminium salt, such as XVIII, with $NaBH_4$, $LiAlH_4$, or catalytic hydrogenation, see route (E). Also, the iminium salt can be isomerized to an enamine such as XXI and reduced by catalytic hydrogenation over platinum oxide.

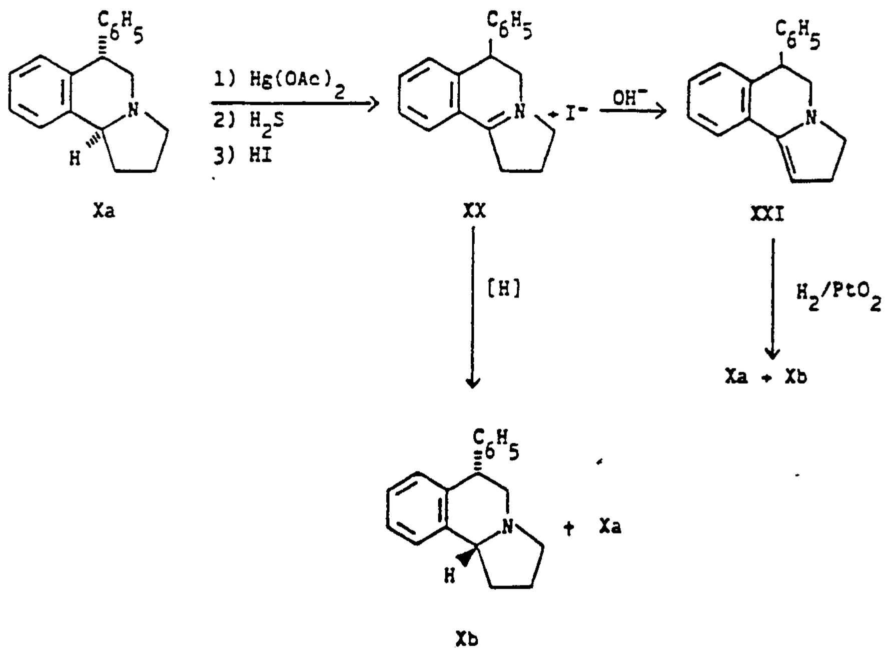

Xa
XX
XXI
Xb

The compounds of this invention can be prepared and utilized in the form of the free base. The compounds can also be used as pharmaceutically-acceptable, nontoxic addition salts of inorganic or organic acids such as halogen acids, e.g., HCl, HBr, HI, sulfuric acid, maleic acid, hexamic acid, perchloric acid, fumaric and saccharin.

Compounds of formula (I) wherein the 6-substituent is hydroxyphenyl were generally prepared from the corresponding methoxy derivatives by standard demethylation using 48% HBr in acetic acid at 100—130°C or $BBr_3$, in methylene chloride at −78 to −20°C. Compounds of formula (I) wherein the 6-substituent is aminophenyl were obtained from nitrophenyl congeners by reduction of the nitro compound with hydrogen in the presence of a catalyst, such as $PtO_2$, in ethanol. From the aminophenyl derivative, the compound wherein $R_8$ or $R_9$ is lower acylamino or benzoylamino may be prepared by reaction with the desired alkanoyl chloride or benzoyl chloride, respectively as described in the Examples. The acylamino derivative may be reduced with $BH_3$ to yield a compound wherein $R_8$ or $R_9$ is alkylamino, e.g., by reducing the compound wherein the 6-substituent is acetylamino ($CH_3CONH$—) substituted phenyl to one wherein the 6-substituent is ethylaminophenyl. The compounds of the invention wherein the 6-substituent is phenyl substituted by cyano may be obtained by reaction of the corresponding bromo compound with cuprous cyanide with tetrakistriphenyl-phosphine palladium and from such a cyano product, the corresponding carboxamide, e.g., $R_8$ = —$CONH_2$ may be obtained by reaction with a strong base such as potassium hydroxide.

The formula (I) compounds are endowed with useful biological activity in the central nervous system. More particularly, the formula (I) compounds exhibit antidepressant activity in mammals. In the group of formula (I) compounds, useful antidepressant activity may be associated with particular diastereomers.

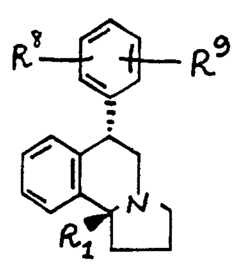

XXII

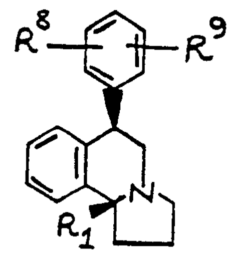

XXIII

The utility of the novel compounds of this patent is based on a standard test for antidepressant agents involving antagonism of the depressant effects of tetrabenazine (TBZ). This is the "classical" tetrabenazine antagonism assay described in US—A—3,787,577. In this test, mice are injected with a test compound 30 minutes prior to the injection of 32 mg/kg i.p. of TBZ, a drug which decreases normal exploratory activity and induces parameters: the presence of normal exploratory activity (EA) and reversal of ptosis (Pt). A control group of mice is given only 32 mg/kg i.p. of TBZ. The biological activity of the novel compounds of this invention may be understood by some representative, nonlimiting examples, presented in Tables I and II.

Table I lists the compounds tested by a compound number, structural formula, HX or acid addition salt form in which the compound was tested, test data, and internal code number (McN No.). Table II names the various Table I compounds.

TABLE 1††

| No. | Compound | HX | TBZ $ED_{50}$* EA/Pt (i.p.) | McN No. (m.p.; solv.)† |
|---|---|---|---|---|
| IVb | 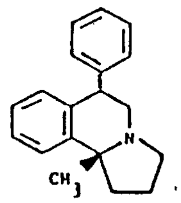 | hexamic acid | 9.2/3.8 | 4803 (136—38; P/EE) |
| Xb | 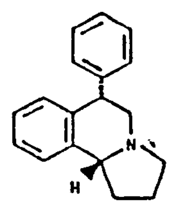 | fumaric acid | 0.34/0.07 | 4612-Z (170—72; E) |
| XXIV | 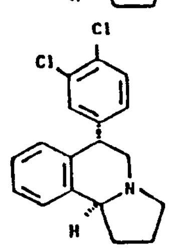 | fumaric acid | CA. 40/8.0 | 5531 (199—205 d; E/W) |
| XXIX | 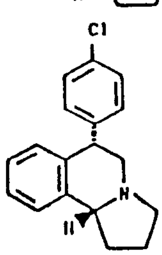 | HBr | 0.27/0.13 | 5292 (290—93; M) |

TABLE 1††

| No. | Compound | HX | TBZ $ED_{50}$* EA/Pt (i.p.) | McN No. (m.p.; solv.)† |
|---|---|---|---|---|
| XXX | 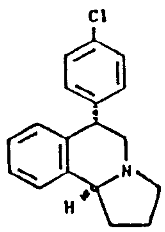 | fumaric acid | 8.1/5.4 | 5321 (179—81; M.P.) |
| XXXI | 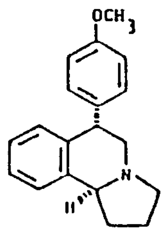 | HBr | ca. 60/3.0 | 5344 (202—204; M/P) |
| XXXIII | 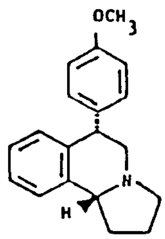 | HBr | 7.0/0.19 | 5346 (241—44; M/P) |
| XXXV | 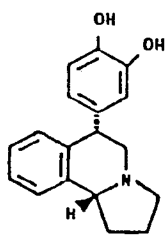 | HBr | 1.4/0.13 | 5375 (244—48 d; B) |
| XXXVI | 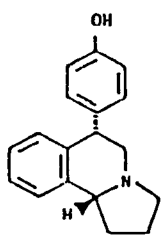 | HBr | ca. 14/10 | 5394 (257—60; M/P) |
| XXXVII | 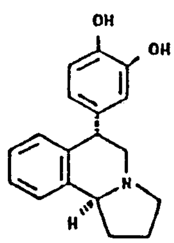 | HBr | 30/1.8 | 5416 (246—48 d; M/B) |
| XXXVIII | 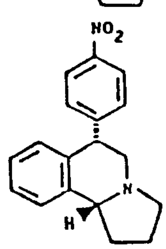 | HBr | 0.51/0.15 | 5462 (227—29 d; E/W) |

TABLE 1††

| No. | Compound | HX | TBZ $ED_{50}$* EA/Pt (i.p.) | McN No. (m.p.; solv.)† |
|---|---|---|---|---|
| XL | 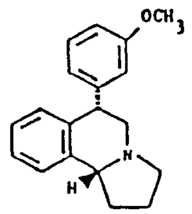 | fumaric acid | ca. 0.17/ ca. 0.07 | 5494 (185—91; P) |
| XLI | 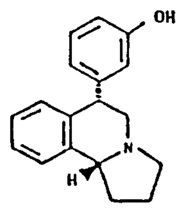 | HBr | >10/0.11 | 5497 (244—46; P) |
| XLIV | 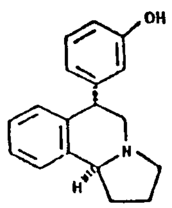 | HBr | 37% @ 30/3.1 | 5498 (252—53; E/EE) |
| XLV | 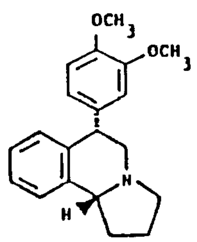 | HBr | 33% @ 30/0.14 | 5426 (235—37 d; A) |
| XLVIII | 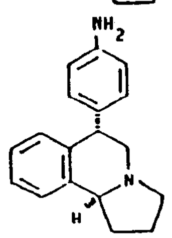 | HBr | 1.4/0.41 | 5556 (220—245 d; M/P) |
| XLIX | 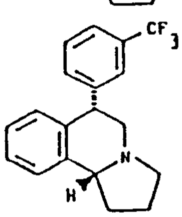 | HCl | 1.2/0.43 | 5558 (180—83 d; D/E) |
| LI | 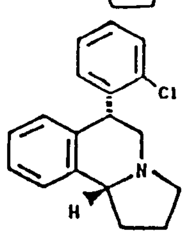 | HBr | 37% @ 60/0.20 | 5707 (213—220; M/P) |

*$ED_{50}$ given in mg/kg for each parameter (except for percent inhibition at a certain dose in mg/kg, given for three examples: XLIV, XLV and LI).

Footnotes continued:

†m.p. of the acid-solution salt in °C (d = decomposition); recrystallization solvent (E = absolute ethanol; M = methanol, P = 2-propanol; EE = ethyl ether; EA = ethyl acetate; B = t-butanol; W = water; A = acetonitrile; Ac = acetone; D = dichloromethane; T = tetrahydrofuran).

††Structure assignments are based mainly on $^1$H NMR spectral data. X-ray crystallographic analyses were performed on XXV, XLVI, the lactam precursor to XXVIII, and the lactam precursor to X*a* (i.e., IX*a*) to establish stereochemistry for the entire series.

TABLE II

| No. | McN No. | Name of compound |
|---|---|---|
| IVb | 4803 | [6α,10bβ]-1,2,3,5,6-10b-Hexahydro-10b-methyl-6-phenylpyrrolo[2,1-a]isoquinoline |
| Xb | 4612-Z | [6α,10bβ]-1,2,3,5,6,10b-Hexahydro-6-phenyl-pyrrolo[2,1-a]isoquinoline |
| XXIV | 5531 | 6α-(3,4-Dichlorophenyl)-1,2,3,5,6,10bα-hexahydro-pyrrolo[2,1-a]isoquinoline |
| XXIX | 5292 | [6α,10bβ]-6-(4-Chlorophenyl)-1,2,3,5,6,10b-hexahydropyrrolo[2,1-a]isoquinoline |
| XXX | 5321 | 6α-(4-Chlorophenyl)-1,2,3,5,6,10bα-hexahydro-pyrrolo[2,1-a]isoquinoline |
| XXXI | 5344 | 1,2,3,5,6,10bα-Hexahydro-6α-(4-methoxyphenyl)-pyrrolo[2,1-a]isoquinoline |
| XXXIII | 5346 | 1,2,3,5,6,10bβ-Hexahydro-6α-(4-methoxyphenyl)-pyrrolo[2,1-a]isoquinoline |
| XXXV | 5375 | 4-(1,2,3,5,6,10bβ-Hexahydropyrrolo[2,1-a]-isoquinolin-6α-yl)benzene-1,2-diol |
| XXXVI | 5394 | 4-(1,2,3,5,6,10bα-Hexahydropyrrolo[2,1-a]-isoquinolin-6α-yl)phenol |
| XXXVII | 5416 | 4-(1,2,5,6,10bα-Hexahydropyrrolo[2,1-a]-isoquinolin-6α-yl)-1,2-benzenediol |
| XXXVIII | 5462 | 1,2,3,5,6,10bβ-Hexahydro-6α-(4-nitrophenyl)-pyrrolo[2,1-a]isoquinoline |
| XL | 5494 | 1,2,3,5,6,10bβ-Hexahydro-6α-(3-methoxyphenyl)pyrrolo[2,1-a]isoquinoline |
| XLI | 5497 | 3-(1,2,3,5,6,10bβ-Hexahydropyrrolo[2,1-a]-isoquinolin-6α-yl)phenol |
| XLIV | 5498 | 3-(1,2,3,5,6,10bα-Hexahydropyrrolo[2,1-a]-isoquinolin-6α-yl)phenol |
| XLV | 5426 | 1,2,3,5,6,10bβ-Hexahydro-6α-(3,4-dimethoxyphenyl)pyrrolo[2,1-a]isoquinoline |
| XLVIII | 5556 | 4-(1,2,3,5,6,10bβ-Hexahydropyrrolo[2,1-a]-isoquinoline-6α-yl)benzenamine |

TABLE II (continued)

| No. | McN No. | Name of compound |
|---|---|---|
| XLIX | 5558 | 1,2,3,5,6,10bβ-Hexahydro-6α-[(3-trifluoromethyl)phenyl]pyrrolo[2,1-a]isoquinoline |
| LI | 5707 | [6α,10bβ]-6-(2-chlorophenyl)-1,2,3,5,6,10b-hexahydropyrrolo[2,1-a]isoquinoline |

Other specific compounds which may be produced include the following:
LIV 1,2,3,5,6,10bα-hexahydro-6α-(4-nitrophenyl)-pyrrolo[2,1-a]isoquinoline;
LV [6α, 10bβ]-6-(4-bromophenyl)-1,2,3,5,6,10b-hexahydropyrrolo[2,1-a]isoquinoline; and
LVI 1,2,3,5,6,10bβ-hexahydro-6α-(4-methylthio-phenyl)-pyrrolo[2,1-a]isoquinoline.

The compound designated LVI was produced through Route (C) and isolated by crystallization in methanol/ethanol as the perchlorate, mp = 202—203.5°C. Preferred compounds of the invention include those designated XLIX, LI and LVI. In general, the 10bβ compounds show greater activity than the corresponding 10bα compounds.

The invention will be further understood by referring to the following examples, which illustrate the preparation of compounds according to the invention. These examples are given for the purpose of illustration only.

The following formula is described hereinafter in Examples 1 and 2.

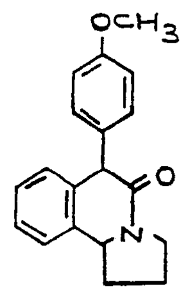

Example 1

Intermediate Lactams

The following procedures for the (A) route refer to the subroutes (a), (b) and (c) shown above.

General Procedure A/(a).

The enelactone (0.11 mol) shown in the scheme, e.g., α-angelica-lactone, in 30 ml of methylene chloride is combined with the arylethylamine, e.g., diphenylethylamine as shown in Route (A), above (0.10 mol) in 30 ml of methylene chloride and let stand for 30 minutes. The solution is evaporated in vacuo to an oil, which is combined with PPA (200 g) and heated on a steam bath for four hours. The reaction mixture is poured into water and extracted with methylene chloride. The organic phase is washed once with water, once with saturated NaCl, and dried ($MgSO_4$). Evaporation in vacuo gives the lactam.

Example A(a)

1,5,6,10b-Tetrahydro-10bα-methyl-6α-phenylpyrrolo[2,1-a]isoquinoline-3(2H)-one (IIIa)

2,3-Diphenylethylamine (6.0 g, 0.03 mol) and α-angelicalactone (3.24 g, 0.003 mol) were combined according to the general procedure. Work-up gave a mixture (94/6, GLC) of diastereomeric lactams IIIa and IIIb. Recrystallization from ethyl acetate/petroleum ether gave white crystalline IIIa, m.p. 135—136°C.

General Procedure A/(b).

Ketoacid (0.10 mol) and dry triethylamine (10.2 g, 0.10 mol) are combined in 50 ml of methylene chloride and cooled to 0°C. Ethyl chloroformate (11.3 g, 0.10 mol) in 25 ml of methylene chloride is added slowly at 0°C to 5°C, stirred at 0°C for two hours and then at 10°C for one hour. 2-Arylethylamine (0.10 mol) in 50 ml of methylene chloride is added to the mixture at 5°C and the solution is stirred overnight at ambient temperature. Water (50 ml) is added and the reaction is stirred for two hours. The organic phase is separated and washed once with 5% HCl, once with 5% $Na_2CO_3$, and dried ($Na_2SO_4$). The solution is filtered and evaporated in vacuo to give the crude keto amide. To this crude material is added PPA (ca. 100 g) and the mixture is stirred on a steam bath for 20 hours. The mixture is poured into water and extracted with chloroform. The organic layer is washed once with $H_2O$, once with 5% $Na_2CO_3$, and dried ($CaCl_2$). Evaporation in vacuo gives crude lactam.

Example A/(b)

1,5,6,10b-Tetrahydro-10bα-methyl-6α-phenylpyrrolo[2,1-a]isoquinoline-3(2H)-one (IIIa)

Following the above procedure, levulinic acid (11.8 g, 0.10 mol) was reacted with 2,2-diphenylethylamine (19.7 g, 0.10 mol) to give a mixture (16/1, GLC) of crude lactams IIIa and IIIb. The mixture was recrystallized from ethyl acetate/hexane to afford white crystalline IIIa, m.p. 177.5—179°C.

General Procedure A/(c)

The keto ester (0.10 mol) and arylethylamine (0.10 mol) are combined and heated at 130°C to 180°C for 24 hours. The oil is placed under vacuum to remove any residual water or ethanol. This residue is combined with PPA (200 ml) and heated on a steam bath for 24 hours. Water (750 ml) is added and solid lactam is filtered or extracted.

Example A(c)

1,5,6,10b-Tetrahydro-10bα-methyl-6α-phenylpyrrolo[2,1-a]isoquinoline-3(2H)-one (IIIa)

2,2-Diphenylethylamine (19.7 g, 0.1 mol) and ethyl levelunate (14.6 g, 0.10 mol) was reacted according to the general procedure to give the lactams mixture IIIa and IIIb (ca. 15/1).

General Procedure B

The arylethylamine (0.10 mol) in 100 ml of dry THF is added slowly to succinic anhydride (0.105 mol) in 100 ml of dry THF at 0°C. (Methylene chloride was also used). The reaction is stirred at ambient temperature for one hour and then evaporated in vacuo to the amide-acid. Cyclization of the amide-acid to the amide is accomplished by either (a) heating it without solvent at 170°C for four hours or (b) combining it with 25 ml of acetyl chloride (AcCl) in 100 ml of ethyl acetate and heating the solution at reflux for ten hours. The imide may be recrystallized before use. The resulting imide is mixed with 400 ml of absolute ethanol, cooled to −10°C with an ice/methanol bath, and stirred efficiently. $NaBH_4$ (0.40 mol) is added followed by 15 drops of $CH_3SO_3H$. The temperature is maintained at −10°C to 0°C with efficient stirring, and five drops of 2 N ethanolic $CH_3SO_3H$ are added every 15 minutes. After five hours, the 2 N $CH_3SO_3H$ is added more rapidly maintaining the temperature at 0°C until the pH is less than three. During this addition, 200 ml of ethanol is added to thin out the foamy reaction solution. The reaction is then stirred for 16 hours at ambient temperature. The reaction is treated with water and methylene chloride. The organic solution is separated, washed once with water, once with saturated NaCl, and dried ($MgSO_4$). Evaporation in vacuo gives the ethoxypyrrolidinone. This ester is combined with PPA (100 ml) and heated on a steam bath for six hours (refluxing ethanolic HCl may be used instead of PPA when cyclizing onto electron rich aromatic groups). The reaction is poured into water and extracted with methylene chloride. The organic layer is separated, and washed once with water, once with saturated NaCl, dried ($MgSO_4$), and evaporated in vacuo to give the crude lactam.

Example B1

1,5,6,10bα-Tetrahydro-6α-phenylpyrrolo[2,1-a]isoquinoline-3(2H)-one (IXa)

Following the general procedure, 2,2-diphenylethylamine (19.7 g, 0.10 mol) and succinic anhydride (10.6 g, 0.105 mol, 99% assay) were combined and heated at 175°C to give the imide. After reduction and cyclization with PPA, work-up gave white solid lactams IXa and IXb (93/7, GLC). Recrystallization from ethyl acetate/methanol furnished white crystalline IXa, m.p. 204.5—205.5°C.

General Procedure C

The mandelic acid (0.10 mol) is combined with 2-arylpyrrolidine (0.10 mol) in xylenes (300 ml) and heated at reflux under a Dean-Stark trap for 45 hours. The solution is evaporated in vacuo to an oil, which is combined with PPA (20 g) and heated at 100°C with occasional stirring for one hour. The mixture is poured into ice water (400 ml) and extracted with methylene chloride. The organic layer is washed with saturated NaCl, dried ($MgSO_4$), and evaporated in vacuo to give the crude lactam.

Example C

1,2,3,10b-Tetrahydro-6-(4-methoxyphenyl)pyrrolo[2,1-a]isoquinoline-5(6H)-one (6)

4-Methoxymanpelic acid (26 g, 0.133 mol) was reacted with 2-phenylpyrrolidine (21 g, 0.143 mol) to give a brown oil, which was combined with PPA (300 g) and heated at 100°C. Work up furnished the desired lactams as a nearly 3:2 mixture of diastereomers (6a:6b) which were separated by HPLC.

General Procedure D

The styrene oxide (0.10 mol) and 2-aryl-pyrrolidine (0.10 mol) are combined in absolute ethanol or tetramethylene sulfone (100 ml) and the solution is refluxed for 2 to 8 hours. For ethanol, the solution is evaporated in vacuo to an oil and, for sulfolane, it is diluted with a 5-fold volume of water. The oil is partitioned between ether and 1 N HCl (1 liter). The aqueous solution is made alkaline with 50% NaOH and extracted with ether. The ether solution is dried ($K_2CO_3$) and evaporated in vacuo to an oil, which is combined with polyphosphoric acid (250 g), heated at 100°C for 30 minutes and poured into 1 liter of crushed ice. The mixture is extracted with $CH_2Cl_2$ (4 × 500 ml portions). The organic layer is washed once with 50% NaOH, 3 times with water, dried ($K_2CO_3$), and evaporated in vacuo to give products.

Example D

1,2,3,5,6,10bβ-Hexahydro-6α-[(3-trifluoromethyl)phenyl]pyrrolo[2,1-a]isoquinoline (XLIX)

*m*-Trifluoromethylstyrene oxide (28.0 g, 80% assay, 0.12 mol) and 2-phenyl-pyrrolidine (19.9 g, 88% assay, 0.12 mol) were combined in absolute ethanol (120 ml) and treated according to the general procedure to give the crude product mixture (3/1 by GLC). The isomeric amines were separated by preparative HPLC (chloroform/ethyl acetate, 9:1) to give title compound XLIX, which was converted to its HCl salt.

Example 2

Reduction of Lactams

General Procedure

The intermediate lactams, e.g., III, IX and XIV, may be reduced to form the compounds of this invention by careful reduction with borane·THF. The lactam (0.018 mol) is dissolved in THF (40.0 mol) and added slowly to borane·THF (1.0 M, 0.05 mol) at 0°C. The solution is heated at reflux for one hour and cooled to 0°C. Water (10.0 ml), followed by hydrochloric acid (12.0 M, 15.0 ml), are added (both slowly). The reaction is stirred for two hours at ambient temperature. The THF is distilled off, 50 ml of water is added, and the solution is heated at reflux for 15 minutes. The solution is cooled in an ice bath, made alkaline with 1 N NaOH, and extracted with methylene chloride. The organic phase is washed once with water, once with saturated NaCl, and dried ($K_2CO_3$). The solution is evaporated in vacuo to give the amine.

Example 3

Equilibration

Equilibration of Lactams. General Procedure

The lactam (0.10 mol) is dissolved in 200 ml of DMSO. Water (20 ml) and $K_2CO_3$ (100 g) are added. The reaction is heated at reflux (generally using an oil bath at 130°C) until equilibrium is reached. This usually required one to three hours of heating. Reaction progress is monitored by removing aliquots, quenching them in ice water, extracting with methylene chloride, and analyzing by GLC. The reaction is rapidly cooled with an ice bath and treated with water (500 ml) and methylene chloride (500 ml). The organic layer is separated, washed three times with water, once with saturated NaCl, and dried ($MgSO_4$). The solution is evaporated in vacuo to give a mixture of lactams, which is generally separated using liquid chromatography.

Example

1,5,6,10bβ-Tetrahydro-6α-phenylpyrrolo[2,1-a]isoquinoline-3(2H)-one (IXb)

The crude mixture (93/7, GLC) of lactams IXa and IXb (19.5 g, 0.075 mol) was combined with 150 ml of DMSO, 15 ml of water and $K_2CO_3$ (7.5 g) according to the general procedure. At equilibrium the reaction was worked up to give a mixture (1/l, GLC) of lactams IXa and IXb (16.0 g, 82%). The lactams were separated using high performance liquid chromatography on a silica gel column. Lactam IXb was recrystallized from ethyl acetate to give white crystals, m.p. 126.5—132.5°C.

Equilibration of Amines. General Procedure

The amine (0.01 mol) is dissolved in 30 ml of DMSO and 30 ml of 10 N NaOH (aqueous) and heated at reflux under $N_2$. When equilibrium is attained, the reaction is quickly cooled in an ice bath and treated with water (200 ml) and methylene chloride (300 ml). The organic phase is separated, washed three times with water, once with saturated NaCl, and dried ($K_2CO_3$). The solution is evaporated in vacuo to give a mixture of the amines.

For pharmaceutical purposes, the compounds according to the present invention are administered to warm-blooded animals enterally or parenterally as active ingredients in customary dosage unit form consisting essentially of an inert pharmaceutical carrier and one effective dosage unit (1—500 mg) of the active ingredient, such as tablets, coated pills, capsules, wafers, powders, solutions, suspensions, emulsions, syrups and suppositories. The daily human dosage range for the treatment of depression is about 10 to 2000 mg of a compound of the invention, e.g., 200 to 500 mg, for an average human. Of course, the exact dosage will vary according to the activity of the particular compound chosen and the weight and need of the patient. Such a dosage may be divided into 2—4 administrations per day.

The following examples illustrate a few pharmaceutical dosage unit compositions comprising a compound of the present invention as an active ingredient and represent suitable modes for putting the invention into practical use. The parts are parts by weight unless otherwise specified.

Example 4

Tablets

The tablet composition is compounded from the following ingredients:

| | |
|---|---|
| 1,2,3,5,6,10bβ-Hexahydro-6α-phenylpyrrolo[2,1-a]isoquinoline (Xb) Fumarate | 100.0 parts |
| Lactose | 45.0 parts |
| Corn Starch | 45.0 parts |
| Colloidal silicic acid | 2.0 parts |
| Soluble starch | 5.0 parts |
| Magnesium stearate | 3.0 parts |
| Total | 200.0 parts |

The active ingredient is admixed with part of the excipients, and the mixture is granulated with a solution of the soluble starch in water. After drying of the granulate, the remaining excipients are admixed with it, and the mixture is compressed into 100 mg tablets. Each tablet contains 50 mg of the pyrroloisoquinoline compound and is an oral dosage unit with effective pharmacologic action.

Example 5

Coated Pills

The pill core composition is compounded from the following ingredients:

| | |
|---|---|
| 1,2,3,5,6,10bβ-Hexahydro-6α-phenylpyrrolo[2,1-a]isoquinoline (Xb) Fumarate | 100.0 parts |
| Lactose | 75.0 parts |
| Corn Starch | 65.0 parts |
| Colloidal silicic acid | 2.0 parts |
| Soluble starch | 5.0 parts |
| Magnesium stearate | 3.0 parts |
| Total | 250.0 parts |

The ingredients are compounded as described in Example 6, and the composition is compressed into 100 mg pill cores which are subsequently coated in a conventional manner with a thin shell consisting essentially of a mixture of sugar, talcum and gum arabic. Each coated pill contains 40 mg of the pyrroloisoquinoline compound and is an oral dosage unit composition with effective pharmacologic action.

Example 6

Syrup

The syrup composition is compounded from the following ingredients:

| | |
|---|---|
| 1,2,3,5,6,10bβ-Hexahydro-6α-phenylpyrrolo[2,1-a]isoquinoline (Xb) Fumarate | 100.0 parts |
| Cane Sugar | 150.0 parts |
| Glycerol (twice distilled) | 250.0 parts |
| Methyl p-hydroxybenzoate | 3.0 parts |
| Propyl p-hydroxybenzoate | 2.0 parts |
| Flavorings, as desired | |
| Water (distilled) | 1.995.0 parts |
| Total | 2,500.0 parts |

Example 7

Tablets

The tablet composition is compounded from the following ingredients:

| | |
|---|---|
| 1,2,3,5,6,10bβ-Hexahydro-6α-(4-nitrophenyl)pyrrolo[2,1-a]isoquinoline (XXXVIII) Hydrobromide | 100.0 parts |
| Lactose | 20.0 parts |
| Corn Starch | 20.0 parts |
| Colloidal silicic acid | 2.0 parts |
| Soluble starch | 5.0 parts |
| Magnesium stearate | 3.0 parts |
| Total | 150.0 parts |

The active ingredient is admixed with part of the excipients, and the mixture is granulated with a solution of the soluble starch in water. After drying of the granulate, the remaining excipients are admixed with it, and the mixture is compressed into 100 mg tablets. Each tablet contains 50 mg of the pyrroloisoquinoline compound and is an oral dosage unit with effective pharmacologic action.

Example 8

Coated Pills

The pill core composition is compounded from the following ingredients:

| | |
|---|---|
| 4-(1,2,3,5,6,10bβ-Hexahydropyrrolo[2,1-a]-isoquinolin-6α-yl)benzenamine XLVIII) Hydrobromide | 100.0 parts |
| Lactose | 45.0 parts |
| Corn Starch | 45.0 parts |
| Colloidal silicic acid | 2.0 parts |
| Soluble starch | 5.0 parts |
| Magnesium stearate | 3.0 parts |
| Total | 200.0 parts |

The ingredients are compounded as described in Example V, and the composition is compressed into 150 mg pill cores which are subsequently coated in a conventional manner with a thin shell consisting essentially of a mixture of sugar, talcum and gum arabic. Each coated pill contains 75 mg of the pyrroloisoquinoline compound and is an oral dosage unit composition with effective pharmacologic action.

Example 9

[6α,10bα]-6-(4-aminophenyl)-1,2,3,5,6,10b-hexahydropyrrolo[2,1-a]-isoquinoline

24.2 g of the nitro compound 1,2,3,5,6,10bα-hexahydro-6α-(4-nitrophenyl)pyrrolo[2,1-a]isoquinoline (LIV) in 600 ml of ethanol is treated with 2.5 g of $PtO_2$ and warmed to dissolve the substrate, if necessary. The mixture is hydrogenated at 45 psig for about 45 minutes. Filter aid (3 g) is added and the reaction is filtered. The solution is evaporated to a light tan syrup, which eventually crystallizes on prolonged standing. The monohydrobromide salt, prepared in 2-propanol with 48% HBr, is recrystallized from methanol to give light tan leaflets, mp 252—258°C.

Example 10

[6α,10bα]-6-(4-acetylaminophenyl)-1,2,3,5,6,10b-hexahydropyrrolo[2,1-a]-isoquinoline

To 2.0 g of the aniline product of Example 9 in 15 ml of dry $CH_2Cl_2$ was slowly added 650 mg of acetylchloride in 5 ml of $CH_2Cl_2$, with stirring. After 16 hours, the acetylchloride reaction was basified with 1N aqueous NaOH and the organic layer was separated. The aqueous layer was extracted and the combined $CH_2Cl_2$ solution was dried ($Na_2SO_4$) and concentrated to a tan solid. Recrystallization from ethyl acetate gave tan leaflets, mp 175—178°C.

Example 11

[6α,10bα]-6-(4-benzoylaminophenyl)-1,2,3,5,6,10b-hexahydropyrrolo[2,1-a]isoquinoline

To 2.0 g of the aniline product of Example 9 in 15 ml of dry $CH_2Cl_2$ was added slowly 1.0 g of benzoylchloride in a 5 ml of $CH_2Cl_2$ with stirring. After 16 hours, the benzoylchloride reaction was diluted with dry ether, cooled to 0°C, and filtered to give a gray solid. The solid was recrystallized from methanol to give a white powder, mp 261—274°C.

Example 12

[6α,10bα]-6-(4-ethylamino)-1,2,3,5,6,10b-hexahydropyrrolo[2,1-a]isoquinoline

To 2.6 g of the amide produced in Example 10 in 20 ml of dry THF was slowly added 30 ml of 1M $BH_3$-THF at 5°C under an inert atmosphere. The reaction was refluxed for 1 hour, cooled in ice and treated with 6 ml of $H_2O$ and then 9 ml of 12N HCl. The THF was distilled off and the reaction refluxed an additional 10 minutes. The solution was ice bath cooled and 3N NaOH was added until the pH was greater than 11 and then extracted with methylene chloride. The organic layer was dried ($K_2CO_3$) and evaporated in vacuo to an oil. The fumarate salt was prepared from methanol/2-propanol to give white crystalline material (1.95 g), mp 170—172°C.

Example 13

[6α,10bβ]-6-(4-cyanophenyl)-1,2,3,5,6,10b-hexahydropyrrolo[2,1-a]isoquinoline

35.2 g (0.107 mole) of the bromide, a mixture of [6α,10bα]- and [6α,10bβ]-6-(4-bromophenyl)-1,2,3,5,6,10b-hexahydropyrrolo[2,1-a]isoquinoline produced via the styrene-oxide route (D), 19.21 g (0.215 mole) of cuprous cyanide and 1.0 g of tetrakistriphenylphosphine palladium (O) were combined in $10^7$ ml of N,N-dimethylacetamide under an inert atmosphere. The reaction was refluxed for 18 hours, cooled and partitioned between 1 liter of conc. ammonium hydroxide and 250 ml of ether. The aqueous layer was extracted several times with ether and the combined ether solution was washed 2 times with ammonium hydroxide, 2 times with water, 3 times with brine, dried ($K_2CO_3$) and evaporated in vacuo to an oily product (22.6 g, 77%). The α and β isomers were separated by preparative HPLC and the 10bβ isomer was isolated. The 10bβ HCl salt was recrystallized from methanol to give an off-white solid, mp 271—276°C.

Example 14

[6α,10bβ]-6-(4-carboxamidophenyl)-1,2,3,5,6,10b-hexahydropyrrolo[2,1-a]isoquinoline

200 mg of the oily cyano compound produced in Example 13 as the 10bα and 10bβ isomer mixture was combined with 150 mg of KOH in 1 ml of t-butanol and refluxed for 30 minutes. The cooled solution was

extracted between methylene chloride and saturated NaCl. The organic layer was dried ($K_2CO_3$) and evaporated in vacuo to a glassy material. The glassy material was then subjected to preparative HPLC to separate the α and β isomers whereby the 10bβ isomer was isolated.

## Claims

1. A compound of the general formula I

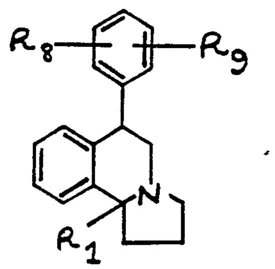

wherein:

$R_1$ is hydrogen or $C_1$—$C_4$ alkyl;

$R_8$ and $R_9$ are independently hydrogen, $C_1$—$C_6$ alkyl, perfluoro $C_1$—$C_4$ alkyl, hydroxy, $C_1$—$C_4$ alkoxy, carb ($C_1$—$C_4$) alkoxy, $C_1$—$C_5$ acylamino, benzoylamino, cyano, carboxamido, $C_1$—$C_5$ acyl, $C_1$—$C_4$ alkylthio, $C_1$—$C_4$ alkylsulfonyl, nitro, amino, $C_1$—$C_4$ alkyl- or di($C_1$—$C_4$) alkyl-amino, or halogen;

including diastereomers and the nontoxic, pharmaceutically-acceptable acid addition salts thereof.

2. A compound of Claim 1, wherein:

$R_8$ and $R_9$ are independently hydrogen, $C_1$—$C_6$ alkyl, perfluoro ($C_1$—$C_4$) alkyl, hydroxy, $C_1$—$C_4$ alkoxy, carb ($C_1$—$C_4$) alkoxy, $C_1$—$C_5$ acylamino, $C_1$—$C_5$ acyl, $C_1$—$C_4$ alkylthio, $C_1$—$C_4$ alkylsulfonyl, nitro, amino, $C_1$—$C_4$ alkyl- or di($C_1$—$C_4$) alkyl-amino, or halogen.

3. A compound of any one of claims 1 or claim 2, which is in the form of 10bβ.

4. A compound of Claim 1, which is the free base or acid addition salt form of:
[6α,10bβ]-1,2,3,5,6,10b-hexahydro-10b-methyl-6-phenylpyrrolo[2,1-a]isoquinoline;
[6α,10bβ]-1,2,3,5,6,10b-hexahydro-6-phenylpyrrolo[2,1-a]isoquinoline;
[6α-(3,4-dichlorophenyl)-1,2,3,5,6,10bα-hexahydropyrrolo[2,1-a]isoquinoline;
[6α,10bβ]-6-(4-chlorophenyl)-1,2,3,5,6,10b-hexahydropyrrolo[2,1-a]isoquinoline;
6α-(4-chlorophenyl)-1,2,3,5,6,10bα-hexahydropyrrolo[2,1-a]isoquinoline;
1,2,3,5,6,10bβ-hexahydro-6α-(4-methoxyphenyl)pyrrolo[2,1-a]isoquinoline;
4-(1,2,3,5,6,10bβ-hexahydropyrrolo[2,1-a]isoquinolin-6α-yl)benzene-1,2-diol;
1,2,3,5,6,10bβ-hexahydro-6α-(4-nitrophenyl)pyrrolo[2,1-a]isoquinoline;
1,2,3,5,6,10bβ-hexahydro-6α-(3-methoxyphenyl)pyrrolo[2,1-a]isoquinoline;
3-(1,2,3,5,6,10bβ-hexahydropyrrolo[2,1-a]isoquinolin-6α-yl)phenol;
1,2,3,5,6,10bβ-hexahydro-6α-(3,4-dimethoxyphenyl)pyrrolo[2,1-a]isoquinoline;
4-(2,3,5,6,10bα-hexahydropyrrolo[2,1-a]isoquinolin-6α-yl)benzenamine;
1,2,3,5,6,10bβ-hexahydro-6α-[(3-trifluoromethyl)phenyl]pyrrolo[2,1-a]isoquinoline;
[6α,10bβ]-6-(2-chlorophenyl)-1,2,3,5,6,10b-hexahydropyrrolo[2,1-a]isoquinoline; or
1,2,3,5,6,10bβ-hexahydro-6α-(4-methylthio-phenyl)pyrrolo[2,1-a]isoquinoline.

5. A compound of Claim 1, which is the free base or acid addition salt of 1,2,3,5,6,10bβ-hexahydro-6α-[(3-trifluoromethyl)phenyl]pyrrolo[2,1-a]isoquinoline.

6. A compound of Claim 1, which is the free base or acid addition salt of [6α,10bβ]-6-(2-chlorophenyl)-1,2,3,5,6,10b-hexahydropyrrolo[2,1-a]isoquinoline.

7. A compound of Claim 1, which is the free base or acid addition salt of 1,2,3,5,6,10bβ-hexahydro-6α-(4-methylthiophenyl)pyrrolo[2,1-a]isoquinoline.

8. A compound of Claim 1, which is the free base or acid addition salt of 1,2,3,5,6,10bα-phenylpyrrolo[2,1-a]isoquinoline.

9. A pharmaceutical composition comprising a compound of any one of claims 1 to 8 in combination with a pharmaceutically acceptable diluent or carrier.

10. A compound of any one of claims 1 to 8 or a composition of claim 9 for use in treating depression in a mammal.

**Patentansprüche**

1. Verbindung der allgemeinen Formel I

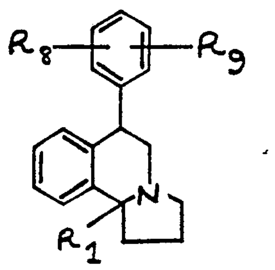

(I)

worin:

$R_1$ für Wasserstoff oder $C_1$—$C_4$Alkyl steht; und

$R_8$ und $R_9$ unabhängig voneinander Wasserstoff, $C_1$—$C_6$Alkyl, Perfluor $C_1$—$C_4$alkyl, Hydroxy, $C_1$—$C_4$Alkoxy, Carb($C_1$—$C_4$)alkoxy, $C_1$—$C_5$Acylamino, Benzoylamino, Cyano, Carboxamido, $C_1$—$C_5$Acyl, $C_1$—$C_4$Alkylthio, $C_1$—$C_4$Alkylsulfonyl, Nitro, Amino, $C_1$—$C_4$Alkyl- oder Di($C_1$—$C_4$)alkylamino oder Halogen bedeuten,

einschließlich der Diastereomeren und der nichttoxischen, pharmazeutisch annehmbaren Säureadditionssalze hievon.

2. Verbindung nach Anspruch 1, worin:

$R_8$ und $R_9$ unabhängig voneinander Wasserstoff, $C_1$—$C_6$Alkyl, Perfluor $C_1$—$C_4$alkyl, Hydroxy, $C_1$—$C_4$Alkoxy, Carb($C_1$—$C_4$)alkoxy, $C_1$—$C_5$Acylamino, $C_1$—$C_5$Acyl, $C_1$—$C_4$Alkylthio, $C_1$—$C_4$Alkylsulfonyl, Nitro, Amino, $C_1$—$C_4$Alkyl- oder Di($C_1$—$C_4$)alkylamino oder Halogen bedeuten.

3. Verbindung nach einem der Ansprüche 1 oder 2, welche in Form der 10bβ-Verbindung vorliegt.

4. Verbindung nach Anspruch 1, welche in Form der freien Base oder des Säureadditionssalzes von
[6α,10bβ]-1,2,3,5,6,10b-Hexahydro-10b-methyl-6-phenylpyrrolo[2,1-a]isochinolin;
[6α,10bβ]-1,2,3,5,6,10b-Hexahydro-6-phenylpyrrolo[2,1-a]isochinolin;
[6α-(3,4-Dichlorphenyl)-1,2,3,5,6,10bα-hexahydropyrrolo[2,1-a]isochinolin;
[6α,10bβ]-6-(4-chlorphenyl)-1,2,3,5,6,10b-hexahydropyrrolo[2,1-a]isochinolin;
6α-(4-Chlorphenyl)-1,2,3,5,6,10bα-hexahydropyrrolo[2,1-a]isochinolin;
1,2,3,5,6,10bβ-Hexahydro-6α-(4-methoxyphenyl)pyrrolo[2,1-a]isochinolin;
4-(1,2,3,5,6,10bβ-Hexahydropyrrolo[2,1-a]isochinolin-6α-yl)benzol-1,2-diol;
1,2,3,5,6,10bβ-Hexahydro-6α-(4-nitrophenyl)pyrrolo[2,1-a]isochinolin;
1,2,3,5,6,10bβ-Hexahydro-6α-(3-methoxyphenyl)pyrrolo[2,1-a]isochinolin;
3-(1,2,3,5,6,10bβ-Hexahydropyrrolo[2,1-a]isochinolin-6α-yl)phenol;
1,2,3,5,6,10bβ-Hexahydro-6α-(3,4-dimethoxyphenyl)pyrrolo[2,1-a]isochinolin;
4-(2,3,5,6,10bα-hexahydropyrrolo[2,1-a]isochinolin-6α-yl)benzolamin;
1,2,3,5,6,10bβ-Hexahydro-6α-[(3-trifluoromethyl)phenyl]pyrrolo[2,1-a]isochinolin;
[6α,10bβ]-6-(2-Chlorphenyl)-1,2,3,5,6,10b-hexahydropyrrolo[2,1-a]isochinolin; oder
1,2,3,5,6,10bβ-Hexahydro-6α-(4-methylthio-phenyl)pyrrolo[2,1-a]isochinolin vorleigt.

5. Verbindung nach Anspruch 1, welche in Form der freien Base oder eines Säureadditionssalzes von 1,2,3,5,6,10bβ-Hexahydro-6α-[(3-trifluormethyl)phenyl]pyrrolo[2,1-a]isochinolin vorliegt.

6. Verbindung nach Anspruch 1, welche in Form der freien Base oder des Säureadditionssalzes von [6α,10bβ]-6-(2-Chlorphenyl)-1,2,3,5,6,10b-hexahydropyrrolo[2,1-a]isochinolin vorliegt.

7. Verbindung nach Anspruch 1, welche in Form der freien Base oder des Säureadditionssalzes von 1,2,3,5,6,10β-Hexahydro-6α-(4-methylthiophenyl)pyrrolo[2,1-a]isochinolin vorliegt.

8. Verbindung nach Anspruch 1, welche in Form der freien Base oder des Säureadditionssalzes von 1,2,3,5,6,10bα-Phenylpyrrolo[2,1-a]isochinolin vorliegt.

9. Pharmazeutische Zusammensetzung, umfassend eine Verbindung nach einem der Ansprüche 1 bis 8 in Kombination mit einem pharmazeutisch annehmbaren Verdünnungsmittel oder Träger.

10. Verbindung nach einem der Ansprüche 1 bis 8 oder Zusammensetzung nach Anspruch 9 zur Verwendung in der Behandlung von Depressionen bei einem Säuger.

**Revendications**

1. Composé de formule générale I

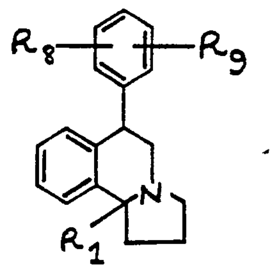

(I)

où:

$R_1$ est un hydrogène ou un alcoyle en $C_{1-4}$; et

$R_8$ et $R_9$ représentent indépendamment un hydrogène, alcoyle en $C_{1-6}$, perfluoro-alcoyle en $C_{1-4}$, hydroxy, alcoxy en $C_{1-4}$, carb-alcoxy en $C_{1-4}$, acylamino en $C_{1-5}$, benzoylamino, cyano, carboxamido, acyle en $C_{1-5}$, alcoylthio en $C_{1-4}$, alcoyle en $C_{1-4}$ sulfonyle, nitro, amino, alcoyle en $C_{1-4}$- ou dialcoyle en $C_{1-4}$-amino, ou halogène,

y compris leurs diastéréomères et sels d'addition d'acides non toxiques pharmaceutiquement acceptables.

2. Composé de la revendication 1, où:

$R_8$ et $R_9$ représentent indépendamment un hydrogène, alcoyle en $C_{1-6}$, perfluoro-alcoyle en $C_{1-4}$, hydroxy, alcoxy en $C_{1-4}$, carb-alcoxy en $C_{1-4}$, acylamino en $C_{1-5}$, acyle en $C_{1-5}$, alcoylthio en $C_{1-4}$, alcoyle en $C_{1-4}$ sulfonyle, nitro, amino, alcoyle en $C_{1-4}$- ou di-alcoyle en $C_{1-4}$-amino, ou halogène.

3. Composé de l'une quelconque des revendications 1 ou 2, qui est sous la forme de 10bβ.

4. Composé de la revendication 1, qui est sous la forme base libre ou sel d'addition d'acides de:
[6α,10bβ]-1,2,3,5,6,10b-hexahydro-10b-méthyl-6-phénylpyrrolo[2,1-a]isoquinoléine;
[6α,10bβ]-1,2,3,5,6,10b-hexahydro-6-phénylpyrrolo[2,1-a]isoquinoléine;
[6α-(3,4-dichlorophényl)-1,2,3,5,6,10bα-hexahydropyrrolo[2,1-a]isoquinoléine;
[6α,10bβ]-6-(4-chlorophényl)-1,2,3,5,6,10b-hexahydropyrrolo[2,1-a]isoquinoléine;
6α-(4-chlorophényl)-1,2,3,5,6,10bα-hexahydropyrrolo[2,1-a]isoquinoléine;
1,2,3,5,6,10bβ-hexahydro-6α-(4-méthoxyphényl)pyrrolo[2,1-a]isoquinoléine;
4-(1,2,3,5,6,10bβ-hexahydropyrrolo[2,1-a]isoquinoléin-6α-yl)benzène-1,2-diol;
1,2,3,5,6,10bβ-hexahydro-6α-(4-nitrophényl)pyrrolo[2,1-a]isoquinoléine;
1,2,3,5,6,10bβ-hexahydro-6α-(3-méthoxyphényl)pyrrolo[2,1-a]isoquinoléine;
3-(1,2,3,5,6,10bβ-hexahydropyrrolo[2,1-a]isoquinoléine-6α-yl)phénol;
1,2,3,5,6,10bβ-hexahydro-6α-(3,4-diméthoxyphényl)pyrrolo[2,1-a]isoquinoléine;
4-(2,3,5,6,10bα-hexahydropyrrolo[2,1-a]isoquinoléine-6α-yl)benzènamine;
1,2,3,5,6,10bβ-hexahydro-6α-[(3-trifluorométhyl)phényl]pyrrolo[2,1-a]isoquinoléine;
[6α,10bβ]-6-(2-chlorophényl)-1,2,3,5,6,10b-hexahydro-pyrrolo[2,1-a]isoquinoléine; ou
1,2,3,5,6,10bβ-hexahydro-6α-(4-méthylthio-phényl)pyrrolo[2,1-a]isoquinoléine.

5. Composé de la revendication 1, qui est la base libre ou le sel d'addition d'acides de la 1,2,3,5,6,10bβ-hexahydro-6α-[(3-trifluorométhyl)phényl]pyrrolo[2,1-a]isoquinoléine.

6. Composé de la revendication 1, qui est la base libre ou le sel d'addition d'acides de la [6α,10bβ]-6-(2-chlorophényl)-1,2,3,5,6,10b-hexahydropyrrolo[2,1-a]isoquinoléine.

7. Composé de la revendication 1, qui est la base libre ou le sel d'addition d'acides de la 1,2,3,5,6,10bβ-hexahydro-6α-(4-méthylthiophényl)pyrrolo[2,1-a]isoquinoléine.

8. Composé de la revendication 1, qui est la base libre ou le sel d'addition d'acides de la 1,2,3,5,6,10bα-phénylpyrrolo[2,1-a]isoquinoléine.

9. Composition pharmaceutique comprenant un composé de l'une quelconque des revendications 1 à 8 en combinaison avec un support ou diluant pharmaceutiquement acceptable.

10. Composé de l'une quelconque des revendications 1 à 8 ou une composition de la revendication 9 pour application dans le traitement de la dépression chez un mammifère.